# EUROPEAN PATENT APPLICATION

(11) **EP 4 775 128 A1**
(43) Date of publication of application: **15.07.2026**
(21) Application number: 24804573.4
(22) Date of filing: 06.09.2024
(51) Int. Cl.: A61B 3/028, A61B 3/10, A61B 3/103, A61B 3/14

(54) **WEARABLE BINOCULAR OPHTHALMIC INSTRUMENT FOR VISUAL SIMULATION OF OCULAR ABERRATIONS AND METHOD FOR VISUAL SIMULATION OF OCULAR ABERRATIONS**

(30) Priority: 08.09.2023 ES 202330762
(71) Applicant: VOPTICA S.L., 30100 Murcia (ES)
(72) Inventor: ARTAL SORIANO, Pablo, 30100 Murcia Murcia (ES); SOOMRO, Shoaib R., 30100 Murcia Madrid (ES)
(74) Representative: Elzaburu S.L.P.
(86) International application number: PCT/ES2024/070539
(87) International publication number: WO 2025/052018

(57) **Abstract**

Wearable binocular ophthalmic instrument (101, 201, 701, 707, 709, 801, 808, 810) for the visual simulation of ocular aberrations and method for visual simulation of ocular aberrations.

The instrument comprises:
- at least one phase modulator (303, 406, 507, 607, 906) for the simultaneous modulation of two pupils, for performing the correction of ocular aberrations and simulating vision through phase profiles,
- a binocular optical assembly for the presentation of visual stimuli, generation of input and output pupils and measurement of ocular aberrations of any order,
- a processing and control unit (703, 803) for connectivity and processing, and
- at least one image capture device.

The associated method consists of the generation of phase profiles for binocular visual simulation, simultaneous measurement of ocular aberrations, and wired, wireless, and mobile control of the instrument.

## Description

### Field of the Invention

The present invention refers to a wearable binocular ophthalmic instrument for the visual simulation of ocular aberrations for a real-world scene or stimulus image and measurement of ocular aberrations. The present invention also refers to a method for performing visual simulation, measuring ocular aberrations, and enabling mobile control and operation of the instrument.

### Background of the Invention

The quality of the vision perceived by the human eye is fundamentally limited by its optics. Optical aberrations reduce the quality of the image produced on the retina and are one of the major causes of vision problems around the World. Visual simulation is the process of measuring and correcting ocular aberrations by employing optoelectronic techniques, such as adaptive optics. Adaptive optics allows the measurement and correction of aberrations with high precision and in real time. Visual simulation using adaptive optics techniques is usually implemented through digitally controlled phase modulators that are either liquid crystals or deformable mirrors. The ophthalmic instruments providing the capability of aberrations measurements and corrections using adaptive optics are known as visual simulators. One of the early demonstrations of such an instrument was presented in E. J. Fernández, S. Manzanera, P. Piers, P. Artal, "Adaptive optics visual simulator", J. Refrac. Surgery, 18, 634-638 (2002). Later developments in visual simulation instruments demonstrated the measurement and correction beyond low-order aberrations and presented the visual simulation for higher-order aberrations. A key technique that is widely used for the measurement of aberrations utilizes the Hartmann-Shack sensor. Its early use on human eyes was presented by Liang, B. Grimm, S. Goelz, and J. F. Bille, "Objective measurement of WA's of the human eye with the use of a Hartmann-Shack wave-front sensor". J. Opt. Am. A 11, 1949-1957 (1994) and J. Liang and D. R. Williams, "Aberrations and retinal image quality of the normal human eye". J. Opt. Soc. Am. A 14,2873-2883 (1997). To this date, the Hartmann-Shack sensor is broadly used in visual simulators and other relevant ophthalmic instruments for the objective assessment of ocular aberrations.

Document US Pat. No. 7,264,354 B2 reveals an electronically controlled phoropter for visual simulation that incorporates optoelectronic lenses. The optical power of the optoelectronic lenses is controlled by the examiner by varying the electrical signal supplied to the lenses. The presented instrument has the ability to correct defocus only and cannot simulate the correction for astigmatism or other complex aberrations.

Document US Pat. No. 4,943,162 A discloses an instrument that facilitates visual correction using a rotating multi-lense configuration. It presents an instrument that has two stacked cylindrical lenses which rotate symmetrically to compensate for the correction of the subject's spherical defocus and astigmatism.

Document US Pat. No. 8,911,084 B2 discloses a binocular vision simulation instrument that utilizes a phase modulator to correct the aberrations of any type for both eyes simultaneously. The instrument can simulate the corrections for both low-level aberrations and high-level aberrations. The instrument assembly is large and has the form factor of a desktop ophthalmic instrument.

Document US Pat. No. 7,914,148 B2 discloses a method and associated instrument to simulate the optical effect of a selected ophthalmic lens design. The instrument has the form factor of a spectacle and uses an image projector and display screen to show the simulated resultant image.

Document US Pat. No. 5,495,305 A discloses specialized contact lenses that produce visual distortions for the subject to simulate the vision for different visual conditions. The disclosed invention mainly focuses on the visual simulation of distortions and artifacts that may arise after an ocular surgical procedure.

Document US Pat. No. 11,360,313 B1 discloses a binocular system and method for a wearable instrument to automatically correct the spherical refraction in near-eye displays. The invention uses adaptive optics lenses to electronically correct the spherical refraction of the subject for virtual reality and augmented reality near-eye displays.

Document US Pat. No. 10,932,901 B2 discloses a method and associated system that uses a femtosecond pulse laser focused on an optical polymer material or tissue to manipulate its refractive index and demonstrate various types of visual corrections.

Document US Pat. No. 10,898,073 B2 discloses a binocular optoelectronic instrument for the visual simulation and correction in presbyopia. The revealed invention uses optoelectronic lenses and pupil tracking to automatically correct the vision for different viewing distances.

Document US Pat. No. 10,386,645 B2 presents a wearable instrument for testing, identifying, and compensating the ocular pathologies that affect the vision of a patient. The revealed device is spectacle type and has digital screens that provide customized visual field enhancement for the subject.

Document US Pat. No. 8,876,289 B2 discloses an instrument for visual simulation of multifocal corrections. The instrument comprises a combination of a Badal system, beam splitters, and mirrors to allow the simulation of different optical powers for near and far vision.

Document US Pat. No. 7,697.212 B2 presents an invention to simulate the correction and optimization of higher-order aberrations for human eyes when using various optical instruments such as binoculars, telescopes, and microscopes.

Document US Pat. No. 9,681,800 B2 discloses an invention of a see-through adaptive optics phoropter. The instrument comprises a plurality of optical elements that also include an adaptive optics lens, in which the focal power is controlled electronically. The diffractive element is used to relay the light scattered by the human eye to the wavefront sensor.

Since the early demonstration of visual simulation, a range of visual simulation instruments of various modalities has been presented. However, most of the disclosed inventions to this date have presented tabletop instruments that are immobile and bulky in size and require sophisticated handling. On the other hand, the existing wearable-type visual simulation and correction instruments have mainly focused on the correction and simulation of focal power only.

### Summary of the Invention

The object of the invention is to provide a wearable binocular ophthalmic instrument and associated method for the simulation of visual corrections and measurement of ocular aberrations to allow the perception of presented visual stimuli with optical corrections.

The invention presents a wearable binocular ophthalmic instrument for the measurement and visual simulation of ocular aberrations comprising:
- at least one phase modulator for the simultaneous modulation of two pupils, for performing the correction of ocular aberrations and simulating vision through phase profiles,
- a binocular optical assembly for the presentation of visual stimuli, generation of input and output pupils and measurement of ocular aberrations of any order,
- a processing and control unit for connectivity and processing, and
- at least one image capture device.

The invention also presents a method for visual simulation of ocular aberrations, that uses a wearable binocular ophthalmic instrument of the invention, and that comprises the following steps:
- generation of phase profiles for binocular visual simulation,
- simultaneous measurement of ocular aberrations, and
- control and operation of the instrument.

The present invention therefore provides a wearable binocular instrument for simulating the vision corrections for any ocular aberrations that is fully mobile compact, robust, and capable of handling both objective and subjective testing. The instrument can provide realistic visual simulation due to its see-through features. The instrument is highly portable due to its wearable form factor and does have moving components.

The instrument enables the simulation of vision for real-world scenes or stimulus images and also facilitates the simulated vision correction as it would be after an eye is gone through a surgical procedure, such as the implantation of interocular lenses or refractive surgery.

### Brief Description of the Figures

Accompanying figures are solely provided for the purpose of exemplifying the wearable binocular ophthalmic instrument and associated method disclosed in this invention. The figures are not meant to delimit the scope of protection as identified in the claims nor should they be referred to alone in an effort to interpret the scope identified in said claims.
FIG. 1 shows the schematic diagram of a headset-type binocular wearable instrument illustrating an embodiment of the invention.
FIG. 2 shows the schematic diagrams of a spectacle-type binocular wearable instrument illustrating an embodiment of the invention.
FIG. 3 shows the schematic diagram for the layout of the open-view binocular instrument illustrating an embodiment of the invention.
FIG. 4 shows the schematic diagram for the layout of one of the two modules of the close-view binocular instrument illustrating an embodiment of the invention.
FIG. 5 shows the schematic diagram for the layout of the open-view binocular instrument utilizing a pi-phase modulator and double-pass configuration indicating an embodiment of the invention.
FIG. 6 shows the schematic diagram for the layout of one of the two modules of the binocular instrument using a pi-phase modulator and double pass configuration indicating an embodiment of the invention.
FIG. 7A, FIG 7B, and FIG 7C show the schematic diagrams of the open-view binocular instrument variations respectively featuring a wired connection to the control unit, a wireless connection to the control unit, and integrated control unit illustrating different embodiments of the invention.
FIG. 8A, FIG 8B, and FIG 8C show the schematic diagrams of the close-view binocular instrument variations respectively featuring a wired connection to the control unit, a wireless connection to the control unit, and integrated control unit illustrating different embodiments of the invention.
FIG. 9 shows the schematic diagram for the optical layout of the open-view binocular instrument utilizing only one phase modulator for both eyes using active shutters and time-sequential operation illustrating an embodiment of the invention.

### Detailed Description of the Invention

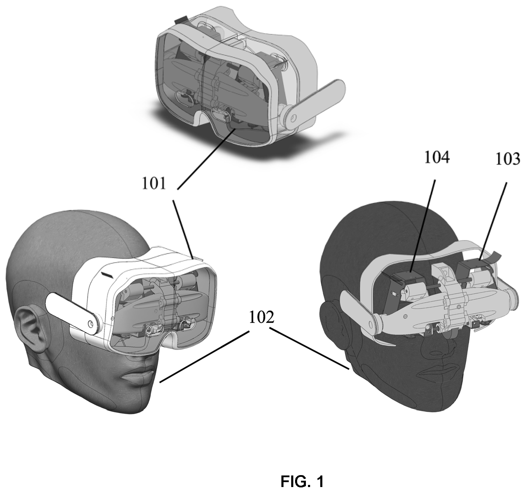

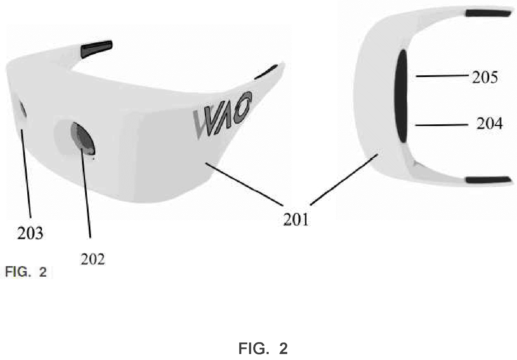

The present invention consists of a wearable binocular ophthalmic instrument for visual simulation and measurement of ocular aberrations of any type, and the method associated with the wearable instrument. The disclosed instrument has close-view and open-view variations with tethered, wireless, and mobile operation capabilities. The disclosed instrument has a wearable form factor that can be in the shape of a head-mounted headset 101 as illustrated in FIG. 1 or a shape like a spectacle 201 as illustrated in FIG. 2.

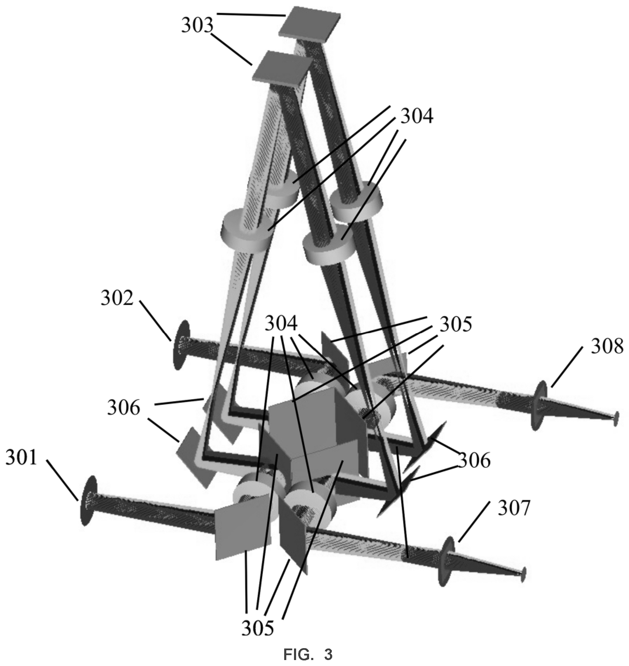

FIG. 3 shows the open-view variation of the wearable binocular instrument that performs the visual simulation of a live real-world view. The open-view wearable instrument includes a pair of fixed-size diaphragms, facing towards the real view, serving as left and right input pupils (301 and 302). A pair of lenses 304 is used to conjugate the input pupil to a phase modulator 303 where phase profiles are induced. The modified pupil then transfers through another lens pair to produce left and right output pupils (307 and 308), which coincide with the subject's left and right eye pupils, respectively. The flat mirrors 306 and prisms 305 are positioned between the lenses to fold the optical path and ensure the see-through arrangement of the instrument.

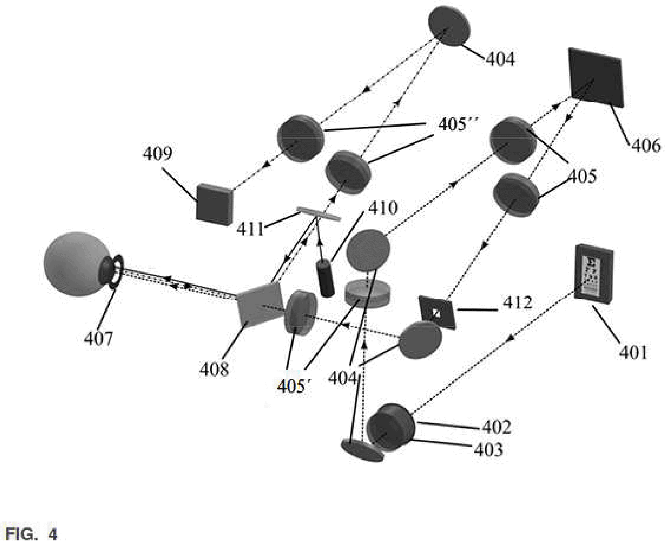

FIG. 4 shows the detailed layout and components of one of the modules of a close-view binocular instrument. The close-view binocular wearable instrument includes a stimuli-presenting screen 401 such as a microdisplay to present the computer-generated stimuli image such as acuity charts. A fixed-sized diaphragm is placed at a predefined distance from the microdisplay which serves as the input pupil 402 of the instrument. A collimating lens 403 is placed immediately after the diaphragm which is used to collimate the light from microdisplay and achieve a predefined field of view. A pair of first small achromatic lenses 405 is then placed in sequence to conjugate the input pupil to the phase modulator 406 where the phase profiles are relayed. The flat mirrors 404 are used in between different optical components to fold the optical path at different locations. The phase modulator 406 is followed by another second pair of achromatic lenses 405' to transfer the modified input pupil and generate the output pupil 407. The focal length of achromatic lenses is chosen such that a predetermined magnification is achieved when conjugating the input pupil to the modulated pupil, and output pupil. A field stop 412 is also placed between the modulated pupil and the output pupil. When wearing the instrument, the subject's eye pupil is aligned with the generated output pupil. The close-view instrument module also contains a wavefront sensor, preferably a Hartmann-Shack sensor 409, a third pair of achromatic lenses 405'', and an illumination source 410 for the objective measurement of the aberrations. Additionally, a hot mirror 408 is fixed before the output pupil that separates the visual and measurement paths. The working wavelength range of the third pair of lenses 405'' also covers the near infrared region. Similarly, the illumination source 410 is also infrared.

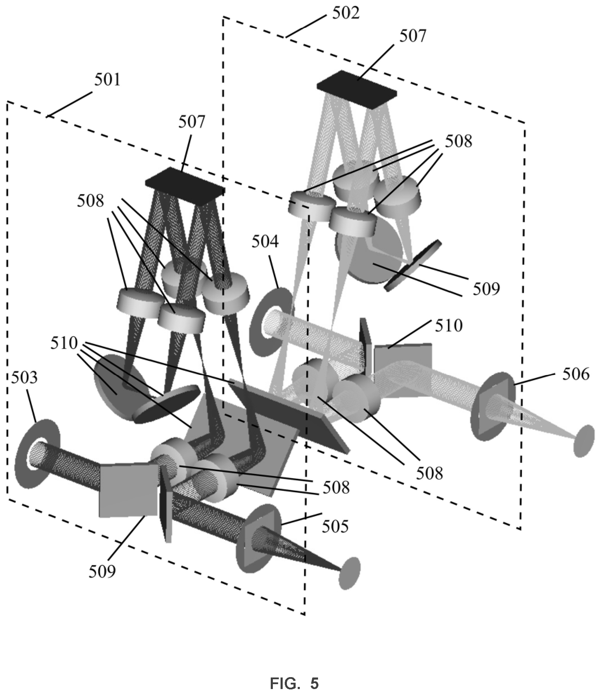

FIG. 5 shows the open-view wearable binocular instrument variation that incorporates a pi phase modulator 507 wherein the optical assembly provides triple conjugation between the input pupil and the output pupil. The instrument contains separate but symmetrical modules (501 and 502) for each of the eyes. Full phase modulation for the complete visual simulation is achieved in two steps wherein a partial pupil modulation is performed in the first step and full phase modulation is achieved in the second phase. A plurality of lenses 508, flat mirrors 510, and prisms 509 are incorporated to perform optical conjugation, fold optical paths and achieve see-through configuration.

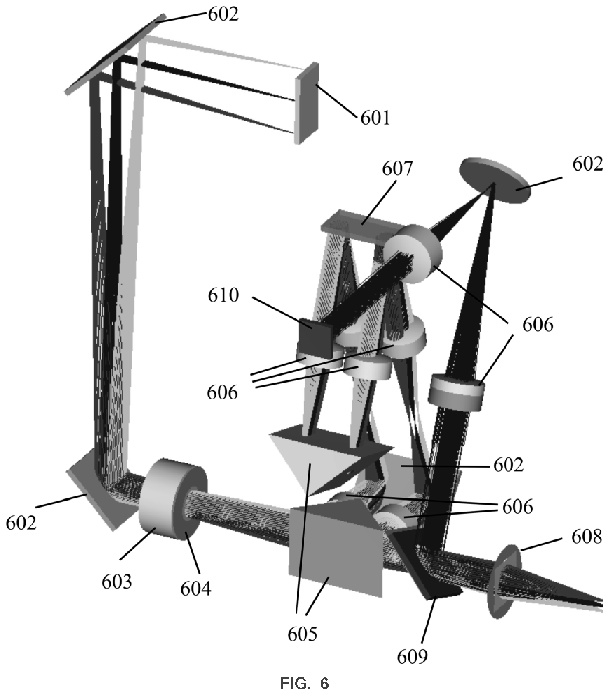

FIG. 6 shows another version of one of the two modules of the close-view binocular instrument that utilizes a pi phase modulator 607 for wavefront manipulation and double-pass pupil configuration for full-phase modulation. This version uses a set of mirrors 602, lenses 606, and prisms 605 in such a configuration that the pupil conjugation is performed two times within the system. The first step of the phase modulation is performed at the first conjugate pupil and the second phase modulation step is completed at the second conjugate pupil where both conjugate pupils utilize different areas of the same phase modulator.

The present invention also provides a method for the visual simulation and measurement of the ocular aberrations that use the binocular wearable instrument presented previously comprising a step of simulating visual corrections, a step of measuring ocular aberrations in binocular settings, and another phase of controlling the operation of the instrument as discussed below:

### Visual simulation:

The subject being corrected wears the binocular instrument ensuring that the subject's eye pupils are aligned with the output pupils of the instrument and the subject's eyes are not accommodating.

The phase profile for the aberration correction is relayed to the panel of the phase modulator which is in conjugation with the input pupil and out pupil, and the incoming stimuli wavefront is manipulated accordingly. The incoming stimuli are either a computed generated image displayed through the means of a microdisplay or a real-world view that is seen through the said instrument.

The modified wavefront is relayed to the respective eye enabling the visual simulation of the applied aberration/correction where the induced aberrations may be identical or different for each of the eyes.

### Aberration measurement:

The measurement of aberrations is performed through either objective or subjective methods.

In the objective method, the aberrations of the subject's eyes are measured through the means of incident light that is first reflected off the retina and is recorded on the wavefront sensor, which is part of the optical assembly of the instrument.

In the subjective method, the aberrations are evaluated by performing the visual simulation for a variety of aberrations, and the visual correction providing the best corrected visual acuity is chosen as a subjectively measured aberration.

### Instrument control and operation:

The wearable instrument is controlled and operated in one of three ways.

The first method is based on the connectivity of the instrument with a control and processing unit such as a computer, tablet or smartphone through a wired interface. The control and processing unit is operated by the examiner, who conducts and monitors the testing procedure, and the phase profile for visual simulation is relayed to the instrument. The overall instrument control and backend processing is managed by the tethered processing and control unit.

The second method is based on the connectivity of the instrument with a control and processing unit through a wireless interface. The control and processing unit is operated by the examiner and the phase profile for visual simulation is relayed to the instrument and overall instrument operation is managed through the wireless processing and control unit.

The third method comprises the use of a compact processing and control unit that is integrated into the wearable instrument. The instrument is operated by the subject itself using an input interface such as touch panel. The phase profiles for visual correction are generated within the instrument and all the processing and control steps are performed on the instrument.

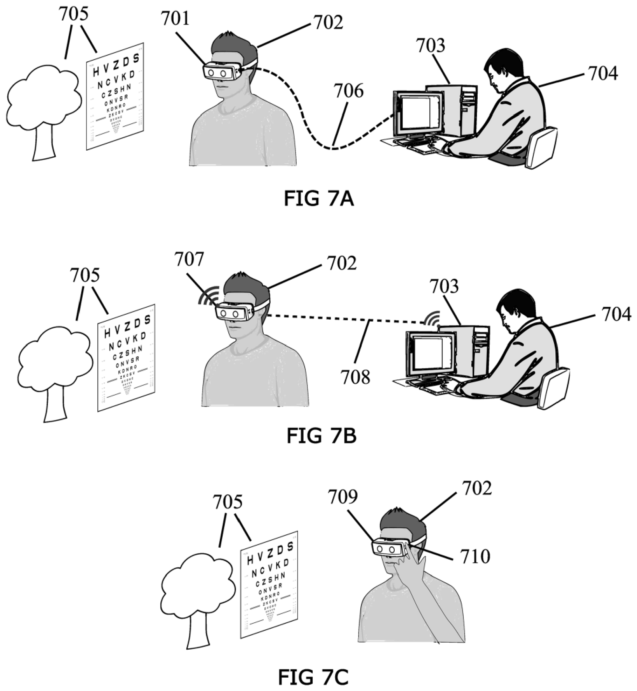

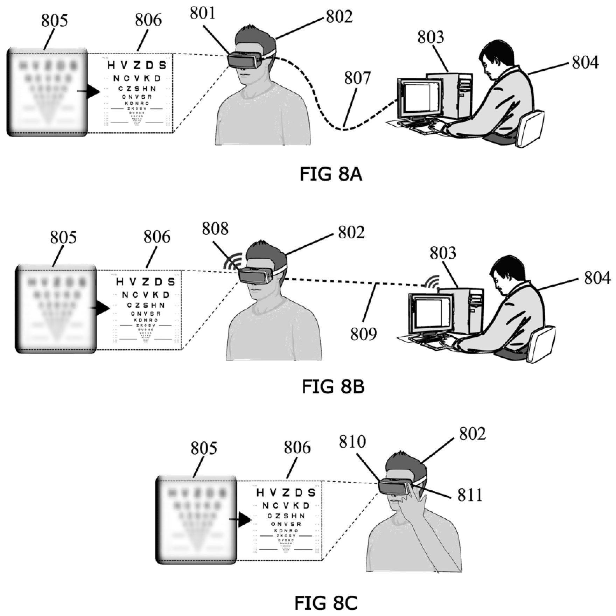

The associated method for control and operation of the instrument is illustrated in FIGS. 7A to 7C and FIGS. 8A to 8C for open-view and close-view instrument variations, respectively. Both instrument variations utilize one of the three different modes of instrument operation. FIG. 7A and FIG. 8A illustrates the connection of the instrument (701 and 801) with the control unit (703 and 803) via a wired interface (706 and 807). FIG. 7B and FIG. 8B illustrates the wireless connectivity (708, 809) between the instrument (707 and 808) and control unit (703 and 803). In both operation modes, the instrument control is managed by the examiner (704 and 804) that performs the vision simulation tests. FIG. 7C and FIG. 8C illustrates the instrument versions (709, 810) in which the control unit is integrated, and all the processing steps are performed within the instrument. Additionally, these instrument variations do not require an examiner and are operated by the subject (702 and 802), through input interface (710 and 811) integrated with the instrument.

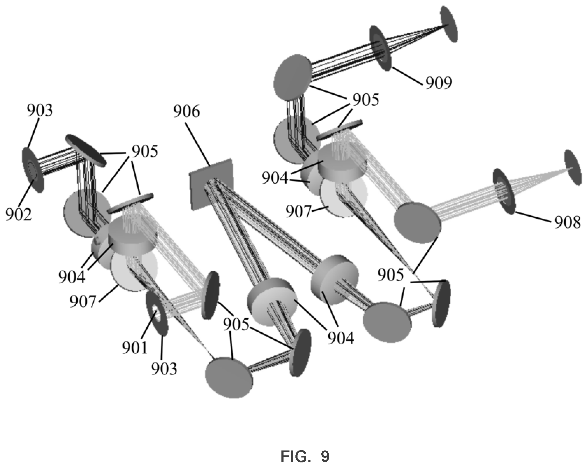

Another version of the open-view instrument layout, shown in FIG. 9, includes single phase modulator 906 along with active shutters 903 and beam-splitters 907 to modulate the left input pupil 901, and right input pupil 902 one by one in a time-sequential pattern. It also includes a lens 904 and a flat mirror 905. A left output pupil 908 and a right output pupil 909 are also shown.

In the wearable binocular ophthalmic instrument of the invention the image capture device is used for imaging the eyes.

The image capture device can be used to perform real-time tracking of eye-pupils.

The image capture device can be a wavefront sensing camera that measures the ocular aberrations.

The instrument of the invention may contain a power source and an energy storage component.

The following numerals are referred to in the detailed description of the present invention:
101- Headset-type binocular instrument
102- Subject under test
103- Left eye module
104- Right eye module
201- Spectacle-type binocular instrument
202- Left input pupil
203- Right input pupil
204- Left output pupil
205- Right output pupil
301- Left input pupil
302- Right input pupil
303- Phase modulator
304- Lens
305- Prisms
306- Flat mirror
307- Left output pupil
308- Right output pupil
401- Stimuli presentation screen
402- Input pupil
403- Collimating lens
404- Flat mirror
405- Lens
405'-Lens
405''-Lens
406- Phase modulator
407- Output pupil
408- Hot Mirror
409- Hartmann-Shack sensor
410- Laser
411- Beam splitter
412- Field stop
501- Left eye module
502- Right eye module
503- Left input pupil
504- Right input pupil
505- Left output pupil
506- Right output pupil
507- Pi Phase modulator
508- Lens
509- Reflective prism
510- Flat mirror
601- Stimuli presentation screen
602- Flat mirror
603- Collimating lens
604- Input pupil
605- Prism
606- Lens
607- Pi-phase modulator
608- Output pupil
609- Hot mirror
610- Hartmann-Shack sensor
701- Open-view binocular instrument with wired connectivity
702- Subject under test.
703- Processing and control unit
704- Examiner
705- Real-world stimuli
706- Tethered connection
707- Open-view binocular instrument with wireless connectivity
708- Wireless interface
709- Open-view binocular instrument with the integrated processing and control unit
710- Input interface for instrument control.
801- Close-view binocular instrument with wired connectivity
802- Subject under test.
803- Processing and control unit
804- Examiner
805- Virtual stimuli aberration-uncorrected
806- Virtual stimuli aberration-corrected
807- Tethered connection
808- Close-view binocular instrument with wireless connectivity
809- Wireless interface
810- Close-view binocular instrument with the integrated processing and control unit
811- Input interface for instrument control.
901- Left input pupil
902- Right input pupil
903- Active shutters
904- Lens
905- Flat mirror
906- Phase modulator
907- Beam splitter
908- Left output pupil
909- Right output pupil

## Claims

1. Wearable binocular ophthalmic instrument (101, 201, 701, 707, 709, 801, 808, 810) for visual simulation of ocular aberrations, **characterized in that** it comprises:
- at least one phase modulator (303, 406, 507, 607, 906) for the simultaneous modulation of two pupils, for performing the correction of ocular aberrations and simulating vision through phase profiles,
- a binocular optical assembly for the presentation of visual stimuli, generation of input and output pupils and measurement of ocular aberrations of any order,
- a processing and control unit (703, 803) for connectivity and processing, and
- at least one image capture device.

2. Wearable binocular ophthalmic instrument (101, 201, 701, 707, 709, 801, 808, 810) according to claim 1,
wherein the said binocular optical assembly creates two input pupils (202, 203; 301, 302; 503, 504; 901, 902) and two output pupils (204, 205; 307, 308; 505, 506; 908, 909) and the said at least one phase modulator (303, 406, 507, 607, 906) is optically conjugated to two input pupils and two output pupils,
wherein the said at least one phase modulator (303, 406, 507, 607, 906) manipulates the phase of two output pupils simultaneously and independently such that the phase manipulation of both output pupils is the same or different from each other,
wherein the optical conjugation of input and output pupils is performed through the binocular optical assembly, that comprises lenses (304, 405, 508, 606, 904) and mirrors (306, 404, 510, 602, 905),
wherein visual simulation of ocular aberrations, ophthalmic elements, and visual conditions is done in binocular configuration, and
wherein the processing and control unit (703, 803) is responsible for the operation and backend processes associated with the instrument (101, 201, 701, 707, 709, 801, 808, 810).

3. Wearable binocular ophthalmic instrument according to claim 1 or 2, wherein the wearable instrument takes the form of a headset (101).

4. Wearable binocular ophthalmic instrument according to claim 1 or 2, wherein the wearable instrument takes the form of a spectacle (201).

5. Wearable binocular ophthalmic instrument according to claim 1 or 2, wherein the wearable instrument is a close-view type (801, 808, 810).

6. Wearable binocular ophthalmic instrument according to claim 5, wherein the stimuli for both eyes are presented on a micro-display screen (401).

7. Wearable binocular ophthalmic instrument according to claim 1 or 2, wherein the instrument is an open-view or see-through type (701, 707, 709).

8. Wearable binocular ophthalmic instrument according to claim 7, wherein the stimuli for both eyes are live real-world views.

9. Wearable binocular ophthalmic instrument according to claim 1 or 2, wherein the at least one phase modulator (303, 406, 507, 607, 906) is digitally controlled.

10. Wearable binocular ophthalmic instrument according to claim 9, wherein the at least one phase modulator (303, 406, 507, 607, 906) is a transmissive liquid crystal, a reflective liquid crystal or a reflective liquid crystal capable of doing pi phase modulation.

11. Wearable binocular ophthalmic instrument according to claim 10 when the at least one phase modulator (303, 406, 507, 607, 906) is a reflective liquid crystal capable of doing pi phase modulation, wherein the at least one phase modulator is used in double-pass pupil conjugation for full-phase modulation.

12. Wearable binocular ophthalmic instrument according to claim 9, wherein a pair of phase modulators is used where each phase modulator is exclusively used for one eye.

13. Wearable binocular ophthalmic instrument according to claim 9, wherein only one phase modulator is used to modulate both eye pupils.

14. Wearable binocular ophthalmic instrument according to claim 13, wherein the phase modulator is sequentially used for both eyes in a time multiplexing configuration.

15. Wearable binocular ophthalmic instrument according to claim 1 or 2, wherein the wearable instrument (707, 808) is wirelessly connected to the processing and control unit (703, 803).

16. Wearable binocular ophthalmic instrument according to claim 1 or 2, wherein the wearable instrument (701, 801) is wired to the processing and control unit (703, 803).

17. Wearable binocular ophthalmic instrument according to claim 1 or 2, wherein the wearable instrument (709, 810) has an integrated input interface and processing and control unit and is operated by the subject.

18. Wearable binocular ophthalmic instrument according to claim 1 or 2, wherein the image capture device is used for imaging the eyes.

19. Wearable binocular ophthalmic instrument set forth in claim 18, wherein the image capture device is used to perform real-time tracking of eye-pupils.

20. Wearable binocular ophthalmic instrument according to claim 18, wherein the image capture device is a wavefront sensing camera that measures the ocular aberrations.

21. Wearable binocular ophthalmic instrument according to claim 1 or 2, wherein the instrument contains a power source and an energy storage component.

22. Method for visual simulation of ocular aberrations, that uses a wearable binocular ophthalmic instrument (101, 201, 701, 707, 709, 801, 808, 810) of claims 1 to 21, that comprises the following steps:
- generation of phase profiles for binocular visual simulation,
- simultaneous measurement of ocular aberrations, and
- control and operation of the instrument.

23. Method for visual simulation of ocular aberrations, according to claim 22, wherein the control and operation of the instrument (101, 201, 701, 707, 709, 801, 808, 810) can use a wired interface, a wireless interface or can be performed by a processing and control unit integrated into the wearable instrument.
